# EUROPEAN PATENT APPLICATION

(11) **EP 1 051 984 A2**
(43) Date of publication of application: **15.11.2000**
(21) Application number: 99122336.3
(22) Date of filing: 09.11.1999
(51) Int. Cl.: A61M 1/00

(54) **A portable snivel inhaler**

(30) Priority: 12.05.1999 KR 9916980; 02.09.1999 KR 9937191
(71) Applicant: G-intek Co. Ltd., Seoul (KR)
(72) Inventor: Suh, Jeong-Joo, Kangdong-ku, Seoul (KR)
(74) Representative: Grosse, Wolfgang, Dipl.-Ing.

(57) **Abstract**

Disclosed is a motor-driven snivel inhaler wherein the same vacuums out snivel from a human nose through a snivel guide member into a snivel storage pipe, so that the snivel of infants, little children, or patients unable to blow their own noses can be disposed of by the present device.

The snivel inhaler of the present invention is hand held, so the same is convenient and safe to use.

## Description

### CROSS-REFERENCES TO RELATED APPLICATIONS

This application is based on application Nos. 99-16980 and 99-37191 filed in the Korean Industrial Property Office on May 12, 1999, and September 2, 1999, respectively, the content of which is incorporated herein by reference.

### BACKGROUND OF THE INVENTION

### (a) Field of the Invention

The present invention relates to a snivel inhaler, more particularly to a portable household motor-driven snivel inhaler for disposing of snivel from infants or patients.

### (b) Description of the Related Art

Usually infants or little children cannot blow their own nose to eliminate snivel. Therefore their snivel is often disposed of with the use of a snivel-inhaling device. The conventional snivel inhaler is operated manually with both hands. Since infants and children often move while the device is used, it is inconvenient to use it with both hands.

### SUMMARY OF THE INVENTION

The present invention, in order to solve the above problem provides a power-driven portable snivel inhaler that can be used with one hand under a constant vacuum, so that the inhaler has more improved convenience, efficiency, and safety.

The present invention to achieve the above purpose presents a snivel inhaler comprises:
a power source operated by a battery and located inside its case;
a power transmitting means for transforming the rotational movement of a motor into the linear reciprocating movement;
a vacuum-generating means connected to the power-transmitting member for forming a vacuum;
more-than-one suction and exhaust valve assembly connected to the vacuum-generating means for discharging the air held in the vacuum-generating means or inhaling the air kept in a snivel storage pipe;
an air passage tube connected to the suction and exhaust valve assembly;
a snivel storage pipe connected with the air passage tube for storing the inhaled snivel;
a snivel guide member coupled to an end side of the snivel storage pipe and inserted into one's nose to thus draw the inhaled snivel into the snivel storage pipe; and
a case wherein the above components, members, and means can be housed and their respective functions are performed;

### BRIEF DESCRIPTION OF THE DRAWINGS

These and other features, aspects, and advantages of the present invention will become better understood with regard to the following description, appended claims, and an accompanying drawings where:
FIG. 1 is a schematic drawing showing main components according to the embodiment of the present invention;
FIG. 2 is an exploded perspective view of the FIG. 1;
FIG. 3 is a partial sectional view of some components to illustrate an air exit/entrance device according to the first embodiment;
FIG. 4 is a perspective view of relevant parts to illustrate the air exit/entrance device according to the first embodiment.;
FIG. 5 is a circuit diagram of the motor circuit according to the first embodiment;
FIG. 6 is an operation process view to illustrate inhalation of snivel according to the first embodiment;
FIG. 7 is a cross sectional view to illustrate an operation system of an air inhalation part in FIG. 6;
FIG. 8 is a cross sectional view to illustrate an operation system of an air exhalation part in FIG. 6;
FIG. 9 is a cross sectional view to illustrate an operation system of the present invention after the snivel is inhaled according to the first embodiment;
FIG. 10 is a cross sectional view to illustrate an operation system of an air inhalation part in FIG. 9;
FIG. 11 is a cross sectional view to illustrate an operation system of an air exhalation part;
FIG. 12 is a partial cross-sectional view to illustrate a mechanical structure helping facilitate an expansion and contraction of the variable piston;
FIG. 13 is a schematic view to illustrate the structure of a snivel inhaler according to the second embodiment;
FIG. 14 is a drawing to illustrate the power transmission system of FIG. 13 in detail;
FIG. 15 is a cross sectional view to illustrate the structure of the suction and exhaust valves shown in FIG. 13.
FIG. 16 is a top view to illustrate the opening/closing members of the suction and exhaust valves shown in FIG. 15 for forming a vacuum;
FIG. 17 is a side view of the FIG. 17;
FIG. 18 is a schematic view to illustrate a snivel inhaler according to the third embodiment of the present invention;
FIG. 19 is a partially enlarged cross-sectional view of principal parts of a snivel inhaler to explain a temporary stop of snivel inhaling; and
FIG. 20 is a drawing illustrating a structure of a snivel guide member extended out from the case.

### DETAILED DESCRIPTION OF THE PREFERRED EMBODIMENTS

Reference will now be made in detail to the present preferred embodiment of the invention, an example of which is illustrated in the accompanying drawings. Wherever possible, the same reference numbers will be used throughout the drawings to refer to the same or like parts.

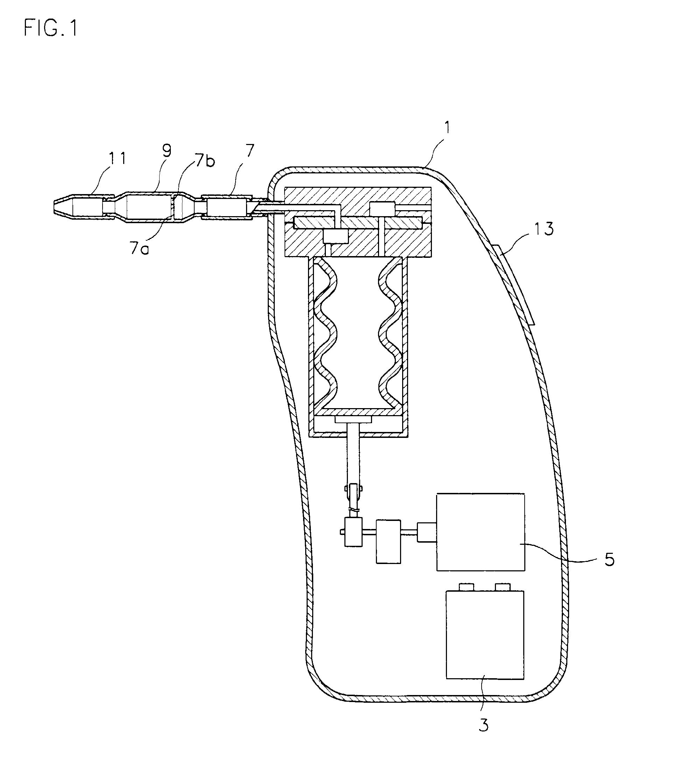

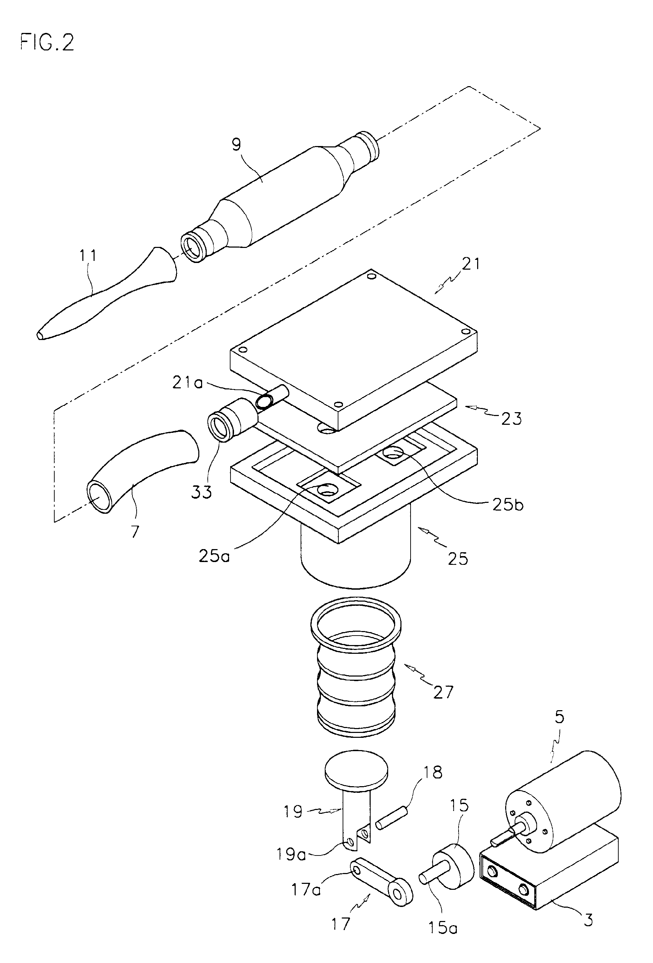

FIG. 1 is an outlined view to illustrate an embodiment of the present invention, and FIG. 2 is an exploded perspective view showing inner parts of the present invention.

The present invention has a case 1 where there is formed enough space accommodating a battery 3, an operating motor 5 connected to the battery, a power transmitting means that conveys the driving power of the motor 5, and a vacuum means where a vacuum is generated by the power transmitting means.

Outside the case is installed an air passage tube 7 connected to the vacuum means, a snivel storage pipe 9 connected to the air passage tube 7, and a snivel guide member 11 for leading snivel into the snivel storage pipe.

A switch 13 for operating the motor 5 is also installed outside the case 1.

The power transmitting means is designed with the following features:
(a) an eccentric cam 15 revolving in accordance with the rotation of the motor 5 is connected to the motor axis.
(b) the rotation center portion of the eccentric cam 15 is in line with the axis of the motor 5, and an eccentric axis 15a is formed in parallel with the axis of the motor spaced apart from the rotation center portion of the eccentric cam 15.
(c) the eccentric axis 15a can be formed having an eccentric member as a unit with the eccentric cam.

The first power transmitting member 17 is rotatably connected onto the outside circumference of the eccentric axis 15a. The first power transmitting member 17 is formed making a right angle with and spaced apart from the eccentric axis 15a. The first power transmitting member 17 has on the other end side thereof opposite to one end where the same is coupled to the eccentric axis 15a a first connection hole 17a through which the second power transmitting member 19 is coupled to the first conveying member 17. On one end of the second member 19 is formed a second connection hole 19a made such that the first connection hole 17a of the first power conveying member 17 is connected to a second connection hole 19a by means of a connection pin 18. The first and second connection holes 17a and 19a of the first and second power conveying members 17 and 19 are positioned so as to overlap and are fixed to each other by the connection pin 18.

The power transmitting means connected to the motor 5 transforms the rotational movement of the motor 5 into the linear reciprocation movement. The other end of the second power transmission member 19 is formed to accomplish a proper connection with a length variable piston and the operation thereof.

The above mentioned vacuum means comprises an air inhalation/exhalation tube member 21, a sealing member 23, a cylinder member 25, and a length variable piston 27.

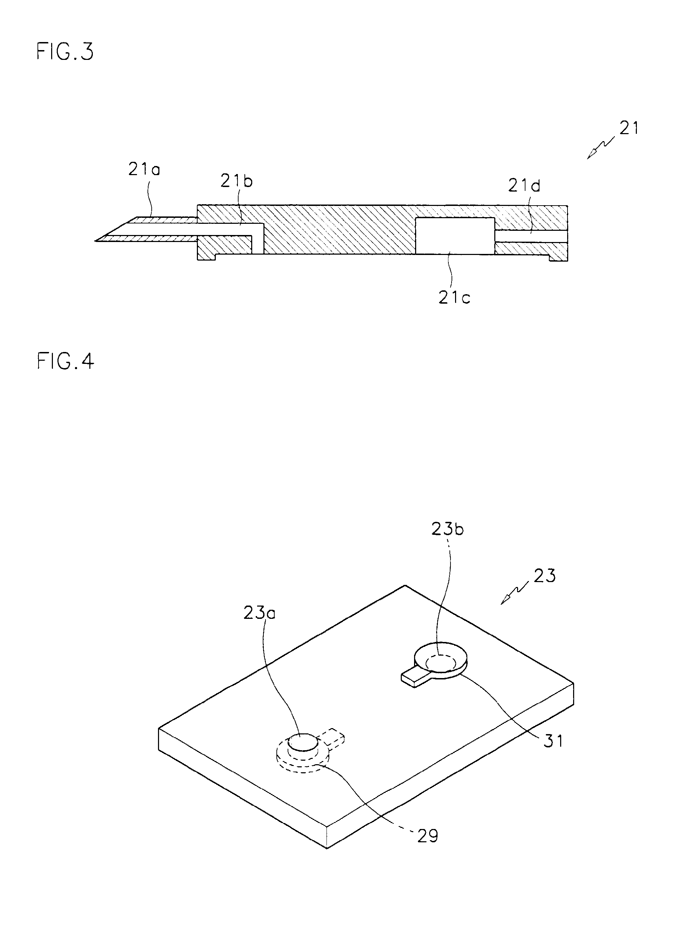

The air inhalation/exhalation tube member 21, as shown in FIG. 3, is formed such that it protrudes from the case 1 having the first inhalation tube 21a wherethrough the air is inhaled. The second inhalation tube 21b is connected to the first inhalation tube 21a inside the air inhalation/exhalation tube member 21. The second inhalation tube 21b has an extended tube portion down the air inhalation/exhalation tube member 21 as illustrated in FIG. 3. The air inhalation/exhalation tube member 21 has a hollow space on one side of the second inhalation tube where a predetermined chamber 21c is made having an exhalation tube 21d connected to the outside of the air inhalation/exhalation tube member 21.

The sealing member 23, as illustrated in FIG. 4, has respective holes 23a and 23b on the portions thereof respectively matching the predetermined chamber 21c connected with the second inhalation tube 21b and the exhalation tube 21d. These portions of sealing member 23 are tightly fixed to the air inhalation/exhalation tube member 21. In the lower end of the hole 23a and in the upper end of the hole 23b are the first and second opening/closing members 29 and 31 used as a check valve couple. The first and second opening/closing members 29 and 31 are preferably made such that they are large enough to cover their respective holes 23a and 23b and composed of a rubber-made elastic body having a disk shape, the first and second opening/closing members being connected to the sealing member 23 with an extended portion thereof as an adhesive, more preferably with an actual adhesive.

The first and second opening/closing members 29 and 31 is designed such that when the air flows in through the air passage tube 7 toward the motor 5, the first opening/closing member hole 23a is opened and the second opening/closing member hole 23b is closed, and when the air is forced to flow from the motor 5 toward the air passage tube 5, the second opening/closing member hole 23b is opened, while the first opening/closing member hole 23a remains closed. When no airflow occurs, the holes are subject to a closing due to resilient elastic action. The sealing member 31 for blocking the inflow of the outside air is made of silicon rubber.

The check valve according to the embodiment of the present invention is not confined to the above-mentioned description. Any check valve having a reversed structural installation for blocking an air inflow in one direction can be applied to the embodiments of the present invention.

The cylinder member 25, whereupon the sealing member 23 and the air inhalation/exhalation tube member 21 are coupled, is formed with a flat faced end so that other parts can be fixed by additional coupling devices.

On this flat faced end is formed other holes 25a and 25b made to match the above holes 23a and 23b.

The cylinder member 25 has a cylindrical member where a predetermined space is formed in the lower end side of the above flat rectangular shape (with reference to FIG. 2). Inside the cylinder member is a variable length piston 27 fixed by insertion.

The upper portion of the variable length piston 27 (with reference to FIG. 2) is coupled to the upper inner surface of the cylinder member 25 with an extra coupling member or an adhesive. The lower portion of the variable length piston 27 is also coupled to the upper portion of the above second power transmitting member 19 (with reference to FIG. 2) with an extra coupling member or an adhesive. The variable length cylinder 27 made of rubber is designed to expand and contract due to its bellows shaped formation.

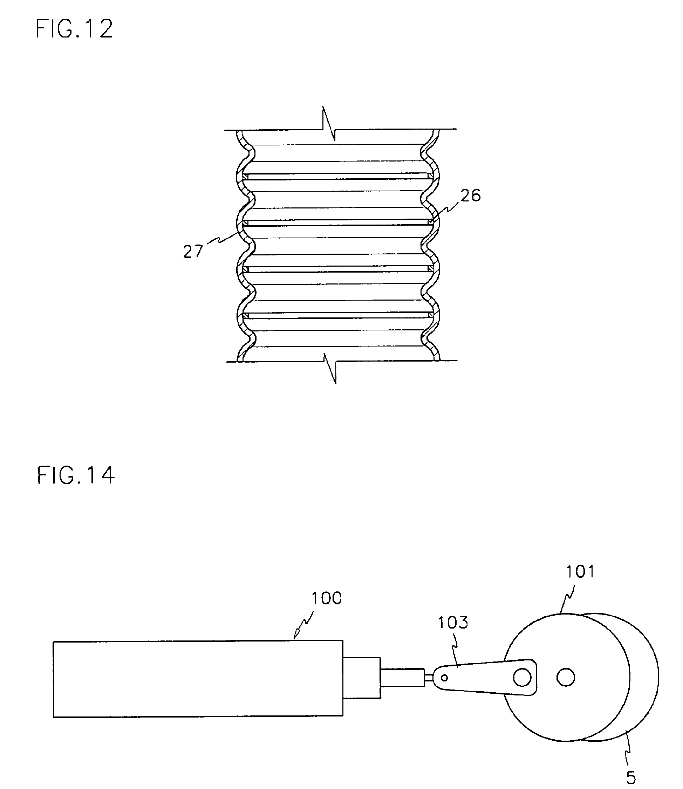

The variable length piston 27 has on its inner circumference elastic members imposing elasticity onto its inner circumference (shown in FIG. 12). The elastic members are to help facilitate the expansion and contraction movement of the variable piston 27, preferably made of piano wire, fishing line, or elastic wire.

One end of the air passage tube 7 is coupled by insertion on the outside circumference of the first inhalation tube 21a which is connected to the air inhalation/exhalation tube 21. In case that the diameter of the air passage tube 7 does not fit when it is inserted onto the second inhalation tube 21b, an extra interface 33 may facilitate the connection.

The air passage tube 7 is preferably made of an elastic rubber.

The other end of the air passage tube 7 is coupled to the snivel storage pipe 9.

The snivel storage pipe 9 has a predetermined volume for storing snivel, a vertical wall 7a in the air passage tube 7 (with reference to FIG. 1) and a passage hole 7b in an upper portion of this vertical wall 7a. The passage hole 7b is formed above the surface of the snivel to thus prevent the influx of snivel into the vacuum means and allow air alone to pass through it.

A snivel guide member 11 is fixed by insertion to the other end of the above snivel storage pipe. The snivel guide member 11 is made in a tube shape and is preferably made of a soft material like rubber because it is directly put into the nose of infants and little children.

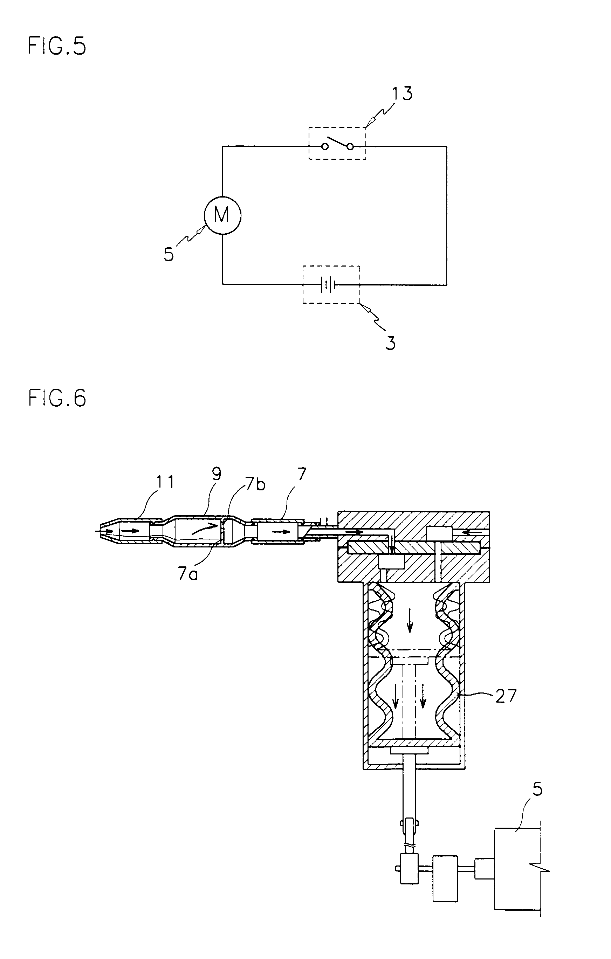

A switch for driving the motor 5 is disposed outside the case 1 and the circuit diagram of its electrical connection is illustrated in FIG. 5.

The switch is simply for conveying battery power to the motor selectively, and if necessary, an electric circuit may be modified using resistors.

A detailed description is made as follows as to how an embodiment of the present invention is operated.

When the switch 13 is turned on after a user inserts into the nose of infants or little children an end portion of the snivel guide member 11, the motor 5 is run by battery power. And the eccentric cam 15 is rotated corresponding to the motor. The rotation movement of the eccentric cam 15 is turned into a linear reciprocation movement through the power transmitting member 17 according to the rotation of the eccentric cam 15. The driving power having a linear movement is transferred to the second power transmitting member 19.

Therefore the second power transmitting member 19 provides a perpendicularly linear reciprocation movement.

When the second power transmitting member 19 makes a downward movement, the variable length piston 27 also makes a downward movement as shown in FIG. 6. At this point, a vacuum is formed inside the variable length piston 27. Snivel in the nose of infants or little children is drawn into the snivel guide member 11 and stored in the snivel storage pipe 9. The snivel stored in the snivel storage pipe is not sent to the vacuum means but remains stored in the snivel storage pipe 9 due to the vertical wall 7a. Only air moves through the passage hole 7b along the arrow-marked direction of FIG. 6.

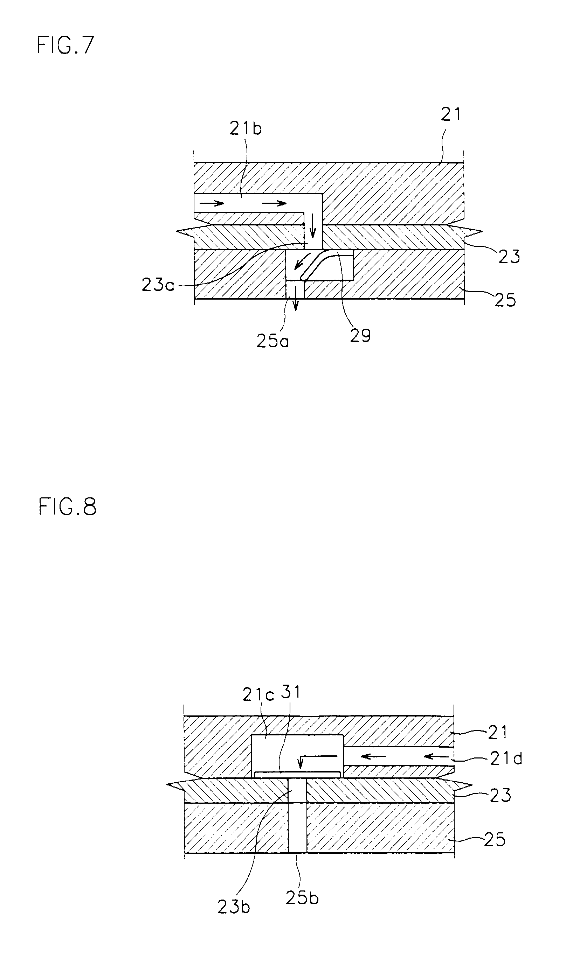

The air is designed to move through the air passage tube 7 into the first inhalation tube 21a and the second inhalation tube 21b. Here the air, as shown in FIG. 7, pushes the first opening/closing member 29 when passing through the hole 23a of the sealing member 23. And the air continues to make its way inside the variable length piston 27 through the cylinder member 25 to thus fill and expand the volume of the length variable piston 27.

While the length variable piston 27 is being extended, the second opening/closing member 31 coupled to the sealing member 23, as displayed in FIG. 8, blocks the inflow of the air.

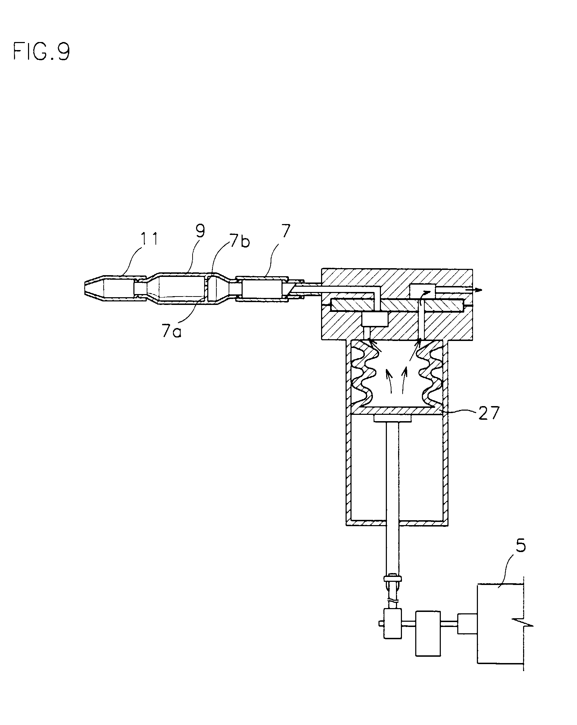

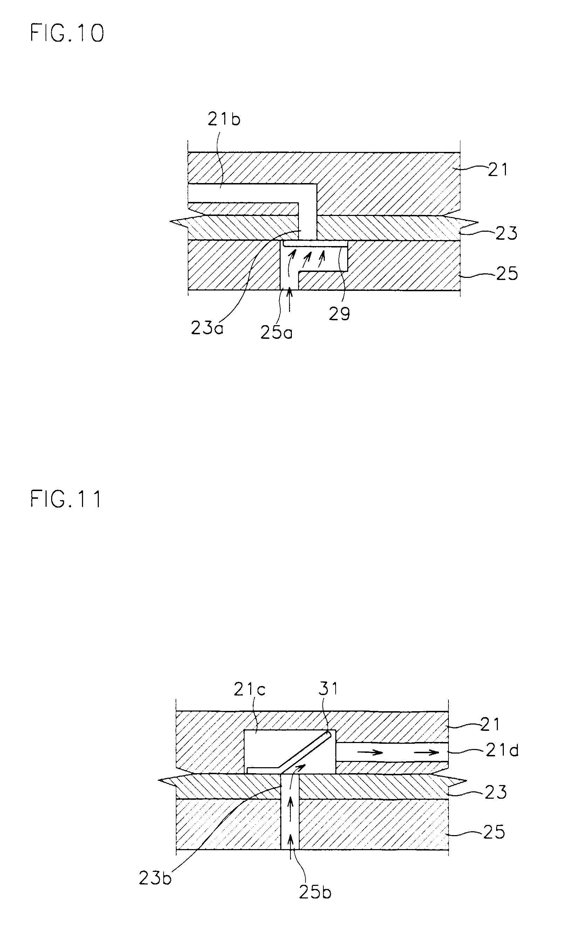

Though the variable length piston, as shown in FIG. 9, is compressed when the second power transmitting member 19 rises, the same returns to its initial position due to air pressure and the elastic power of the first opening/closing member 29, so that the hole 23a of the sealing member 23 is closed (shown in FIG. 10), and the second switching member 31 is pushed up by the air pressure (displayed in FIG. 11), with the result that the air moves along the exhalation tube 21d out into the atmosphere.

Accordingly, snivel is disposed of through the repeated extension/contraction movements of the length variation cylinder 27.

Especially since the snivel guide member 11 and the air passage tube 7 are made of rubber, discomfort to the user's nose is reduced, and the device can operate normally regardless of the variation of the case 1 and of the snivel guide member 11.

Another embodiment of the present invention is described hereinafter in detail.

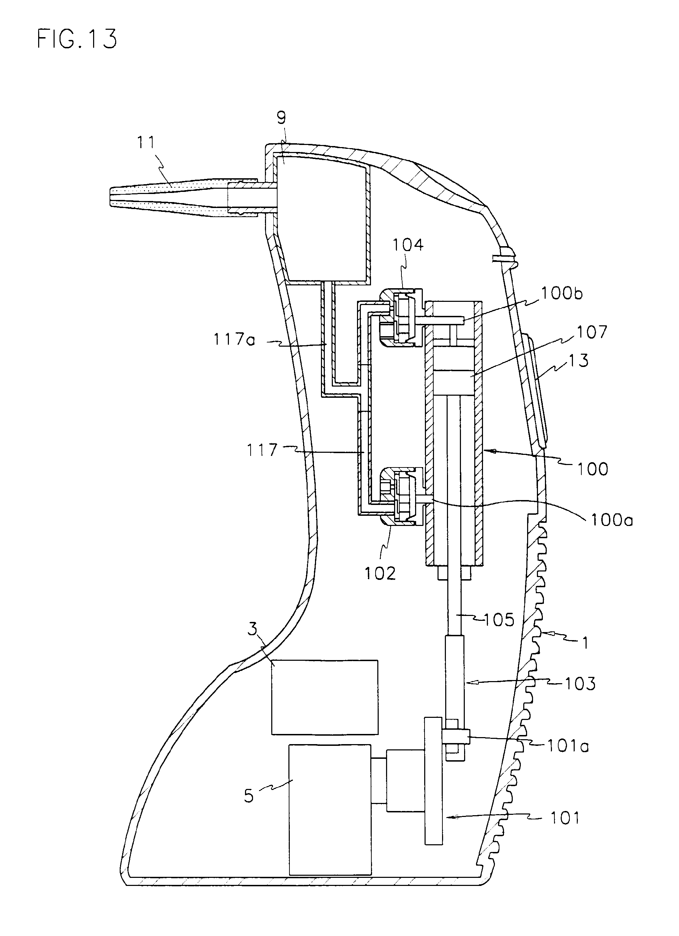

FIG. 13 is a schematic view to illustrate the structure of a snivel inhaler according to the second embodiment of the present invention, which displays a case 1 having an inner space and forming an outer appearance, a battery 3, a motor 5, and a vacuum means mounted in the inner space of the case.

The vacuum means comprises a pair of cylinders 100 for generating air pressure and the suction and exhaust valve assembly 102.

A switch is installed outside the case 1 as was explained in the first embodiment and connected to the case by an electrical circuit formed to run the motor 5 according to a selective transmission of battery power.

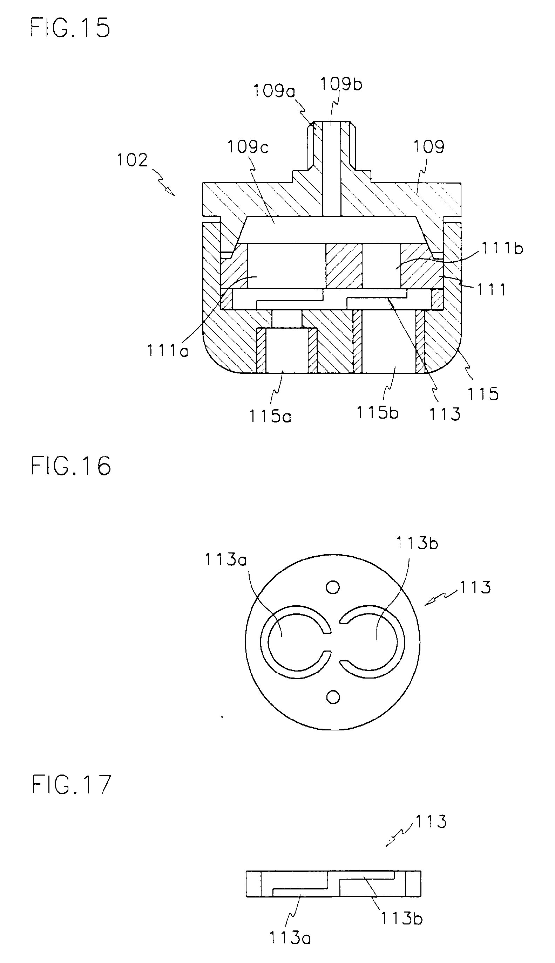

The motor 5 has a disk-shaped rotating member 101 coupled to the axis of the motor. The rotating member 101, as shown in FIG. 14, is designed to rotate along with the rotating axis of the motor 5. And on one edge side of the rotating member 101 is formed an extended arm 101a, preferably located at a point eccentric from the rotating center of the rotating member. In the extended arm 101a is hinged one end of a power transmission member 103 for transforming a rotation movement of the rotating member 101 into a linear movement. The other end of the power transmission member 103 is hinged to a piston rod of the cylinder 100. The piston rod 105 is provided with a piston 107 reciprocating inside the cylinder 100 to thus make a vacuum.

The cylinder 100 has first, and second vacuum ports 100a and 100b connected to the outside to form a vacuum. The first, and the second ports 100a and 100b are preferably disposed outside the stroke of the piston 107 (the movement distance from one end of the reciprocating movement of the piston to the other end thereof)

The first and second ports 100a and 100b have on their inner circumferential surface a thread groove through which the first and second suction and exhaust valve assemblies 102 and 104 are connected.

The first and second suction and exhaust valve assemblies 102 and 104 (for the sake of convenience, we take as an example only the first suction and exhaust valve assembly to explain our purpose), as shown in FIG. 15, comprises:
a connector 109 screwed to the first and second vacuum ports 100a and 100b;
a first suction and exhaust member 111 connected to one end of the connector 109;
an opening/closing member 113 disposed on one end face of the suction and exhaust member 111; and
a second suction and exhaust member 115 having thereinside the first suction and exhaust member 111 and the opening/closing member 113, and connected with the first suction and exhaust member 111.

The connector 109 is made such that one circumferential side of the same has a projected coupling part 109a of a screw thread pattern to which the first and second vacuum ports 100a, 100b are screwed and in the center of the connector is formed an axis-directed air passage 109b.

The other end side opposite the coupling part 109a of the connector 109 has a predetermined spare space, and a screw thread screwed to the second suction and exhaust member 115 is formed on an outer circumference of the spare space. Since inner circumferential surface of the chamber of the connector 109 is formed in a tapered shape, the chamber 109c is still made when the first and second suction and exhaust members are connected. The chamber 109c is designed to facilitate air-flow. The first suction and exhaust member 111 has an air-inhalation hole 111a and an air-exhaust hole 111b and is made such that an outer circumferential surface thereof is formed in a tapered shape corresponding to the tapered part of the connector 109 and that the chamber 109c is secured when the first suction and exhaust member 111 is connected to the connector 109.

The opening/closing member 113 is disposed on one side of the first suction and exhaust member 111 and has the first and second opening/closing parts 113a and 113b as shown in FIG. 16 and 17. The opening/closing part 113 is preferably made of an elastic rubber. One side of the first and second opening/closing parts 113a and 113b formed roughly in a circular shape are connected to the opening/closing member 113. The first opening/closing part 113a is preferably installed to have air exhaust hole 111b subject to selective opening/closing.

The second suction and exhaust member 115 has the first suction and exhaust member 111 and the opening/closing member 113 inside the spare space and is designed such that a screw groove formed in the inner circumferential surface of the spare space is connected to the screw thread.

And the second suction and exhaust member 115 has an air inhalation hole 115a and an air exhaust hole 115b made to be inter-connected with the other end side of the spare space in order to let air pass through. The air inhalation hole 115a is made such that the second opening/closing part 113b of the opening/closing member 113, by contacting the air inhalation hole 115a, opens or closes selectively the air inhalation hole 115a.

The first suction and exhaust valve assembly 102 and the second suction and exhaust valve assembly 104 having identical structures and disposed at each end of the cylinder 100 is structured such that the same 102 and 104 maintain a vacuum consecutively while the piston makes its reciprocation movement. The air passage 109b of the first suction and exhaust valve assembly 102 and the air passage of the second suction and exhaust valve assembly 104 are connected by a conduit 117, and a branch conduit 117a diverged from the conduit 117 is connected to the snivel storage pipe 9. The snivel storage pipe 9 according to the first embodiment is disposed in an outer side of the case 1, but the second embodiment has the snivel storage pipe 9 inside the case 1. However, the snivel storage pipe 9, as in the case of the first embodiment, can be installed outside the case 1 and in other convenient locations.

In one side of the snivel storage pipe 9 is mounted a rubber-made snivel guide member 11, as in the case of the first embodiment.

An operation of a snivel inhaler according to the second embodiment is explained hereinafter.

When the switch is turned on while the snivel guide member 11 is inserted into someone's nose, the battery 3 powered motor 5 rotates a rotating member 101 connected to the motor axis. Then a power transmission member 103, since one side thereof is hinged to an extended arm 101a of the rotating member 101, rotates according to rotation of the rotating member 101. And since the other end of the power transmission member 103 is hinged to the piston rod 105, the rotation movement of the rotating member 101 is changed into the linear movement and therefore, the piston 107 performs a reciprocation movement of a predetermined distance inside the cylinder 100.

A more detailed explanation is made hereinafter about how a vacuum is formed in the snivel storage pipe 9 during the reciprocation movement of the piston 107.

Since the first suction and exhaust valve assembly 102 has the same structure as that of the second suction and exhaust valve assembly 104, an explanation based on the first suction and exhaust valve assembly 102 is made hereinafter.

When the piston 107 moves along the cylinder from the location of the first suction and exhaust valve assembly 102 up to the location of the second suction and exhaust valve assembly 104, a vacuum pressure is formed in the first suction and exhaust valve assembly 102, and the second opening/closing part 113b, by means of the vacuum pressure, opens the air inhalation hole 115a of the second suction and exhaust member 115 to thus form a vacuum inside the snivel storage pipe 9. Then, the air in the snivel storage pipe 9, drawn through the branch conduit 117a and the conduit 117 from the air inhalation hole 115a of the second suction and exhaust member 115 to the air inhalation hole 111a of the first exhaust member, flows into the cylinder 100 through the air passage 109a. Accordingly, the vacuum formed in the snivel storage pipe 9 enables snivel to be inhaled into the snivel storage pipe 9 through the snivel guide member 11.

Reversely, when the piston 107 moves from the second suction and exhaust valve assembly 104 down to the first suction and exhaust valve assembly 102, the second suction and exhaust valve assembly 104 goes through the same operating process as the first suction and exhaust valve assembly 102, and then forms a vacuum inside the snivel storage pipe 9.

A detailed description on this will not be made since given hereinabove.

At this point, the compressed air held at the side of the first suction and exhaust valve assembly 102 of the cylinder 100 is discharged into the atmosphere through an air passage, the air exhaust hole 111b of the first suction member 111, the second suction and exhaust member 115, and then through the air exhaust hole 115b of the second suction and exhaust member 115. This discharge process is completed since, at the same time that the first opening/closing part 113a opened by the compressed air makes an connection with the air exhaust holes 111b and 115b, does the second opening/closing part 113b close the air opening/closing hole 115a by air pressure.

Therefore, as explained above, snivel is inhaled by the vacuum formed inside the snivel storage pipe 9. The snivel storage pipe 9 holds only snivel and lets the air be carried through the conduit 117.

The above mentioned first embodiment has one vacuum state during each stroke of the piston. On the other hand, since the second embodiment of the present invention has two vacuum states during each stroke of the piston, snivel inhalation efficiency is greater than that of the first embodiment due to the more constantly maintained pressure inside the snivel storage pipe.

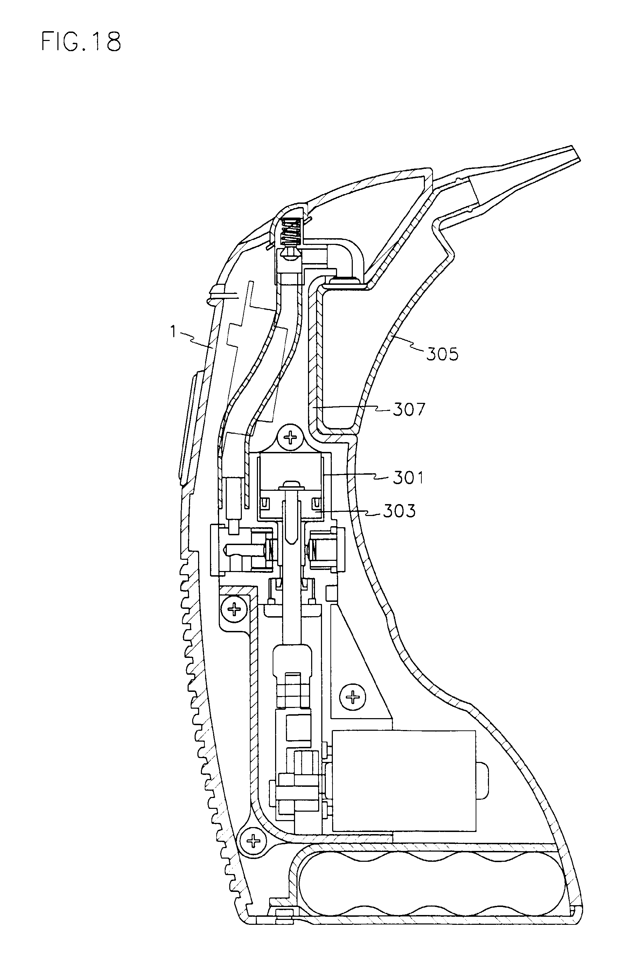

FIG. 18 is a cross sectional view to illustrate a structure of a snivel inhaler according to a third embodiment of the present invention.

A description on the structural and operational differences between the second embodiment and the third embodiment is made hereinafter since the same structural and operational features can be understood with the above explanation of the second embodiment.

A cylinder 301 has one open end and has thereinside a piston making reciprocating movements. And the other end of the cylinder 301, connected to a snivel storage pipe with a conduit to form a vacuum inside the snivel storage pipe, comprises an opening/closing valve and an air exhaust hole wherethrough the air held inside the cylinder is discharged according to the piston 303. The opening/closing valve has the same structure as that of the second embodiment, except that the second embodiment has formed vacuums consecutively due to a pair of opening/closing valves, while the third embodiment, provided with a single opening/closing valve, has a single vacuum generation during a stroke of the piston. The third embodiment which forms a vacuum with a single opening/closing valve can reduce a manufacturing price by decreasing the number of parts.

Another difference is that unlike the second embodiment, the third embodiment has the snivel storage pipe 305 removably connected to the case 1.

That is, an indented portion 307 is formed at one side of the case 1, and then the snivel storage pipe 305 having a corresponding shape to the indented portion 307 is plastically molded, so that the indented portion 307 of the case 1 and the snivel storage pipe 305 are connected by a tight fit.

The snivel storage pipe 305 has a hole connected to a vacuumed conduit, and the case also has a hole connected to a vacuumed conduit so that these two holes may be connected together.

At one side of the vacuum conduit is provided a temporary vacuum stopping means in which snivel inhalation, whenever necessary, is controlled by a vacuum.

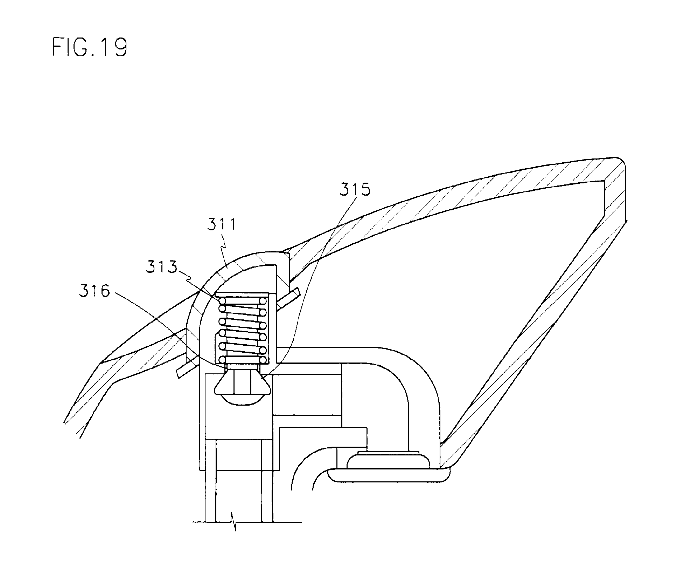

The temporary vacuum stopping means, as shown in FIG. 19, comprises a push-button 311 disposed in the case 1; a compressed coil spring 313 disposed inside the button 311 for returning the button 311 back to its initial location; and a hole stopper member 315 connected to the button 311 for selectively opening or closing a hole made in a vacuum conduit. The hole stopper member 315 disposed inside the vacuum conduit, when the button 311 is pressed, connects the hole formed in the vacuum conduit to the atmosphere, and closes the hole of the vacuum conduit since the button returns to its initial location by the elasticity of the compressed coil spring 313 when the button is not pressed.

Therefore, when a user pushes the button to stop the snivel inhalation temporarily, the vacuum conduit and the atmosphere are connected through the hole 316, with the result that the vacuum is broken.

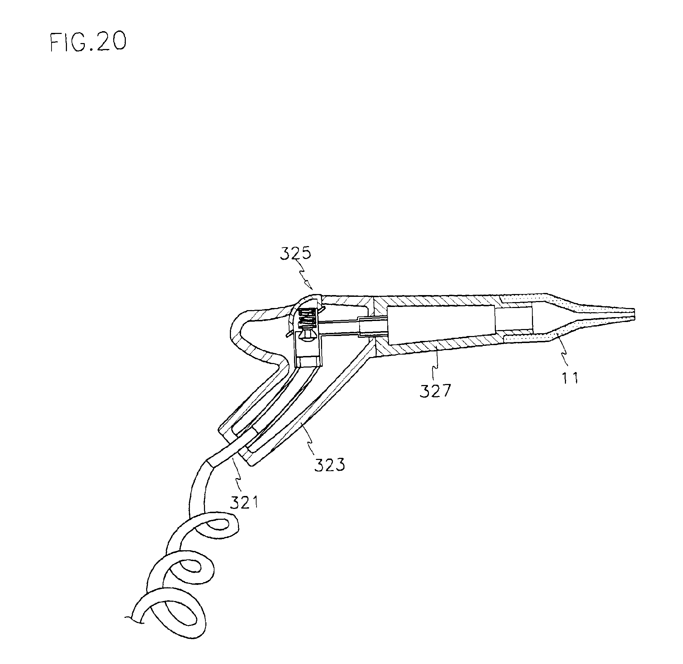

An extended snivel guide member means is illustrated in FIG. 20 in which the snivel storage pipe 305, instead of the snivel guide member, is connected to an extended vacuum conduit 321, which is connected with an auxiliary case 323 where an auxiliary snivel storage pipe 327 and the snivel guide member 11.

In the above auxiliary case 323 is also provided with another temporary vacuum stopping means having the same structure of the temporary vacuum stopping means of the above mentioned third embodiment. Since this temporary vacuum stopping means has the same structure and function as mentioned above, detailed description is replaced with the above explanation. The structure of the temporary vacuum stopping means may also be varied according to a user's preference.

This extended snivel guide member means has an advantage over the first and second embodiments that a user can eliminate snivel at various locations holding only the extended portion while the main case stays at some predetermined place.

The present invention is designed such that the same disposes of snivel from infants or little children with a vacuum formed in its motor-driven vacuum means. The present invention is portable and also usable with one hand, and it has improved safety and efficiency in disposing of snivel and can maintain a constant vacuum pressure due to an enhanced vacuum efficiency.

In this disclosure, there is shown and described only the preferred embodiment of the invention, but, as aforementioned, it is to be understood that the invention is also available in various other combinations and environments and is capable of changes or modifications within the scope of the inventive concepts as expressed in the appended claims.

## Claims

1. A portable snivel inhaler comprising:
a case having a space thereinside;
a driving source operated by a battery and located inside the case;
a power transmitting means for transforming a rotational movement of the driving source into a linear reciprocation movement;
a vacuum generation means connected to the power transmitting means for forming a vacuum;
more-than-one suction and exhaust valve assembly connected to the vacuum generation means for discharging air within the vacuum generation means or inhaling air in a snivel storage pipe;
an air passage tube connected with the suction and exhaust valve assembly;
a snivel storage pipe connected to the air passage tube for storing the inhaled snivel; and
a snivel guide member coupled to an end of the snivel storage pipe and inserted into a user's nose to thus draw snivel into the snivel storage pipe.

2. A portable snivel inhaler according to claim 1, wherein the power transmitting means comprises:
an eccentric cam connected to the axis of the driving source for rotating according to rotation of the driving source and having an eccentric axis at a predetermined distance away from and in parallel with the axis of the rotation of the eccentric cam;
a first power transmitting member perpendicularly connected to the eccentric axis of the eccentric cam for transforming a rotational movement of the driving source into a linear movement; and
a second power transmitting member hinged to the first power transmitting member for making a linear movement in an interlocked connection with the first power transmitting member.

3. A portable snivel inhaler according to claim 1, wherein the driving source is a motor.

4. A portable snivel inhaler according to claim 2, wherein the driving source is a motor.

5. A portable snivel inhaler according to claim 1, wherein the vacuum generation means comprises:
a cylinder having a space thereinside and an air hole through which the suction and exhaust valve assembly inhale air selectively; and
a variable length piston fixed at the inner upper end portion of the cylinder for forming a vacuum during a process of contraction and expansion by a driving power of the power transmitting means.

6. A portable snivel inhaler according to claim 1, wherein the vacuum generation means comprises:
a piston moved by the driving power of the power transmitting means; and
a cylinder disposed at an outside circumference of the piston for forming a vacuum according to the movement of the piston.

7. A portable snivel inhaler according to claim 1, wherein the air passage tube is made of an elastic rubber.

8. A portable snivel inhaler according to claim 1, wherein the suction and exhaust valve assembly comprises:
an air inhalation/exhalation conduit member where an air suction conduit and an air exhaust conduit are formed;
a sealing member coupled with the air suction/exhaust conduit member and having a plurality of check valves connected to the air suction and exhaust conduits for passing or blocking the air selectively.

9. A portable snivel inhaler according to claim 7, wherein a check valve connected to the sealing member comprises;
a first opening/closing portion made with an elastic material and connected on the side of the variable length piston of the sealing member for opening the air suction hole formed in the sealing member when the variable length piston moves down and closing the same when the variable length piston moves up;
a second opening/closing portion made with an elastic material and connected on the opposite side of the variable length piston of the sealing member for closing an exhaust hole formed in the sealing member when the variable length piston moves down and opening the same when the length variable piston moves up.

10. A portable snivel inhaler according to claim 1, wherein the suction and exhaust valve assembly comprises:
a connector connected to one side of the vacuum generation means and having an air passage line communicating with the vacuum generation means;
a first suction and exhaust member disposed at one side of the connector and having an air suction hole and an air exhaust hole;
a second suction and exhaust member housing the first suction and exhaust member and having an air suction hole communicating with the conduit and an exhaust hole for discharging air in the snivel storage pipe, and also connected to the connector; and
an opening/closing member having the first and second opening/closing portions, disposed at a contact face between the first suction and exhaust member and the second suction and closing member, for opening or closing selectively the air exhaust hole of the first suction/exhaust member and the air suction hole of the second suction and exhaust member.

11. A portable snivel inhaler according to claim 9, wherein a plurality of suction and exhaust valve assemblies are mounted on both sides of the vacuum generation means to form a vacuum consecutively according to the vacuum generation means.

12. A portable snivel inhaler according to claim 1, wherein the snivel storage pipe has thereinside a vertical wall for passing only the air through the air conduit into the vacuum means while blocking snivel, and the vertical wall has an air-passing hole placed above the surface level of the stored snivel.

13. A portable snivel inhaler according to claim 10, wherein the suction and exhaust valve assemblies are designed such that the air suction hole of the suction and exhaust valve assembly mounted at one side and the air suction hole of the suction and exhaust valve assembly mounted at the other side are connected by a conduit, and a branch conduit diverged from said conduit is connected to the snivel storage pipe.

14. A portable snivel inhaler according to claim 4, wherein the variable length piston has a plurality of elastic members imposing pressure outwardly against the inner circumferential surface.

15. A portable snivel inhaler according to claim 1, wherein the case has at one side thereof a button operated by a spring for releasing a vacuum held in the snivel storage pipe temporarily and a hole stopper connected to the button for selectively opening or closing a hole connecting the vacuum conduit with the atmosphere.

16. A portable snivel inhaler according to claim 1, wherein the same further comprises:
an extended vacuum conduit connected to one end of the snivel storage pipe;
an auxiliary case connected to the other end of the extended vacuum conduit;
an auxiliary snivel storage pipe connected to the auxiliary case and connected with the extended vacuum conduit; and
a snivel guide member connected to one side of the auxiliary snivel storage pipe.

17. A portable snivel inhaler according to claim 15, wherein the auxiliary case comprises:
a button mounted at one side of the case and operated by a spring for releasing a vacuum held in the snivel storage pipe temporarily;
a hole stopper connected to the button for opening or closing selectively a hole connecting a vacuum conduit and the atmosphere.
